**Europäisches Patentamt**

**European Patent Office**

**Office européen des brevets**

(19)

(11) Veröffentlichungsnummer : **0 314 985 B1**

# EUROPÄISCHE PATENTSCHRIFT

(12)

(45) Veröffentlichungstag der Patentschrift :
13.03.91 Patentblatt 91/11

(51) Int. Cl.$^5$ : **C07C 265/14, C07C 263/10**

(21) Anmeldenummer : 88117456.9

(22) Anmeldetag : 20.10.88

(54) Verfahren zur Herstellung von Mono- und Polyisocyanaten.

(30) Priorität : 31.10.87 DE 3736988

(43) Veröffentlichungstag der Anmeldung :
10.05.89 Patentblatt 89/19

(45) Bekanntmachung des Hinweises auf die
Patenterteilung :
13.03.91 Patentblatt 91/11

(84) Benannte Vertragsstaaten :
**BE DE ES FR GB IT NL**

(56) Entgegenhaltungen :
DE-A- 1 468 445
DE-C- 844 896
FR-A- 2 129 554
US-A- 2 680 127
ULLMANNS ENCYKLOPÄDIE DER TECHNI-
SCHEN CHEMIE, Band 13, Auflage 4, 1977,
Seiten 351-352, New York, US

(73) Patentinhaber : **BAYER AG**
**W-5090 Leverkusen 1 Bayerwerk (DE)**

(72) Erfinder : **Zaby, Gottfried, Dr.**
**Bergische Landstrasse 124b**
**W-5090 Leverkusen (DE)**
Erfinder : **Judat, Helmut, Dr.**
**Oskar-Erbslöh-Strasse 44a**
**W-4018 Langenfeld (DE)**
Erfinder : **Humburger, Siegbert, Dr.**
**Carl-Rumpff-Strasse 8**
**W-5090 Leverkusen (DE)**
Erfinder : **de Vos, Stefaan**
**Vaartlaan 57**
**B-2128 St. Job (BE)**
Erfinder : **Eckermann, Rolf-W.**
**Wichernstrasse 5**
**W-5060 Bergisch-Gladbach (DE)**

EP 0 314 985 B1

## Beschreibung

Die vorliegende Erfindung betrifft ein neues kontinuierliches Verfahren zur Herstellung von Mono- und Polyisocyanaten durch Phosgenierung der ihnen zugrundeliegenden Mono- oder Polyamine, wobei die Umsetzung in speziellen, mit Lochböden ausgerüsteten Kolonnen durchgeführt wird.

Die Herstellung von organischen Isocyanaten durch Phosgenierung der ihnen zugrundeliegenden Amine wurde bisher in Rührkesseln (z.B. DE-OS 1 468 445), Rührkesselkaskaden (z.B. DE-PS 844 896), in mit Füllkörpern gefüllten Reaktionssäulen bzw. -kolonnen (z.B. DE-OS 2 112 181) oder in ungefüllten Kolonnen (z.B. Ullmanns Encyclopädie der technischen Chemie, 4. Auflage (1977), Seiten 351 ff., insbesondere Abbildung 2 auf Seite 352) durchgeführt. Oftmals (beispielsweise beim Verfahren gemäß DE-OS 2 112 181) ist dabei eine Kreislauffahrweise erforderlich, da bei einmaligem Durchgang durch das Reaktionsgefäß kein vollständiger Umsatz erzielt wird.

Jetzt wurde gefunden, daß bei Verwendung von speziellen, mit Lochböden ausgerüsteten Kolonnen, die von dem Reaktionsgemisch von unten nach oben durchströmt werden, eine ganz wesentliche Verbesserung der Phosgenierungsreaktion erzielt werden kann. Das nachstehend näher beschriebene erfindungsgemäße Verfahren zeichnet sich insbesondere durch folgende Vorteile aus :

- Eine Verstopfung des Reaktionsgefäßes findet im Vergleich zu den früher eingesetzten Füllkörperkolonnen nicht statt.
- Im Vergleich zu den bekannten Verfahren unter Verwendung von ungefüllten Kolonnen gestattet das erfindungsgemäße Verfahren wesentlich höhere Raum/Zeit-Ausbeuten bei gleichzeitig niedrigeren Reaktionstemperaturen (Energieersparnis).
- Die Produktausbeute wird trotz vergleichsweise niedrigen Reaktionstemperaturen und Verweilzeiten erhöht.
- Eine Kreislaufführung ist überflüssig, so daß insgesamt einfachere Apparaturen zur Anwendung gelangen.

Gegenstand der Erfindung ist ein Verfahren zur kontinuierlichen Herstellung von organischen Mono- oder Polyisocyanaten in einstufiger Reaktion durch Umsetzung der in organischem Lösungsmittel gelösten, den Mono- oder Polyisocyanaten entsprechenden Mono- oder Polyamine mit in organischem Lösungsmittel gelöstem Phosgen bei erhöhten jedoch unterhalb 150°C liegenden Temperaturen, dadurch gekennzeichnet, daß man das durch kontinuierliche Vereinigung der Aminlösung mit der Phosgenlösung erhaltene Reaktionsgemisch kontinuierlich von unten nach oben eine Reaktionskolonne durchlaufen läßt, welche mindestens 10 durch Lochböden voneinander getrennte Kammern aufweist.

Das erfindungsgemäße Verfahren gestattet die Herstellung von beliebigen organischen Mono- oder Polyisocyanaten durch Phosgenierung der ihnen zugrundeliegenden Mono- oder Polyamine. Als Ausgangsmaterialien kommen beispielsweise folgende Mono- oder Polyamine in Betracht :

Phenylamin, Halogen-substituierte Phenylamine wie 4-Chlorphenylamin, 1,6-Diaminohexan, 1-Isocyanato-3,3,5-trimethyl-5-isocyanatomethyl-cyclohexan, 2,4-Diaminotoluol, dessen technische Gemische mit 2,6-Diaminotoluol oder Polyamingemische der Diphenylmethanreihe, wie sie durch an sich bekannte Anilin-Formaldehyd-Kondensation zugänglich sind.

Besonders bevorzugt wird das erfindungsgemäße Verfahren für die Phosgenierung von 2,4-Diaminotoluol, dessen technische Gemische mit 2,6-Diaminotoluol, die im allgemeinen bis zu 35 Gew.-%, bezogen auf Gemisch, an 2,6-Diaminotoluol enthalten, und von Polyamingemischen der Diphenylmethanreihe verwendet.

Die zu phosgenierenden Amine kommen beim erfindungsgemäßen Verfahren in in inerten Lösungsmitteln gelöster Form zum Einsatz. Diese Aminlösungen weisen im allgemeinen einen Amingehalt von 5 bis 50, vorzugsweise 5 bis 40 und insbesondere 7 bis 20 Gew.-% auf. Bevorzugte Lösungsmittel sind Chlorbenzol oder Dichlorbenzol oder Gemische dieser Lösungsmittel.

Das Phosgen kommt beim erfindungsgemäßen Verfahren in Form von 30- bis 75-, vorzugsweise 30- bis 65- und insbesondere 40- bis 65-gew.-%igen Lösungen in inerten Lösungsmitteln zum Einsatz, wobei vorzugsweise für das Phosgen das gleiche Lösungsmittel wie für das Amin verwendet wird.

Das Äquivalentverhältnis von Amin zu Phosgen liegt im allgemeinen bei 1 : 1,5 bis 1 : 7, vorzugsweise 1 : 2 bis 1 : 5.

Zur Durchmischung der Aminlösung mit der Phosgenlösung können die an sich bekannten Mischaggregate verwendet werden. Hierzu gehören beispielsweise Gegenstrommischkammern (DE-PS 1 146 872), Kreiselpumpen (DE-PS 949 227 oder DE-AS 2 153 268), Venturimischdüsen (DE-AS 1 175 666 oder US-PS 3 507 626), Inlinemischer (US-PS 3 321 283) oder Turbularreaktoren (US-PS 3 226 410).

Das durch Durchmischung der Ausgangslösungen hergestellte Reaktionsgemisch wird beim erfindungsgemäßen Verfahren von unten nach oben durch eine Reaktionskolonne (Phosgenierturm) geleitet, die mit Lochböden ausgerüstet ist, so daß der Innenraum der Kolonne in mindestens 10, vorzugsweise 20 bis 50, durch die horizontal eingebauten Lochböden voneinander abgetrennten Kammern eingeteilt ist. Grundsätzlich möglich, jedoch keinesfalls bevorzugt, wäre auch die Verwendung von mehreren in Serie geschalteten Lochbodenkolonnen mit insgesamt mindestens 10, vorzugsweise 20 bis 50 Kammern.

Eine noch weitergehende Kammerung ist nicht sinnvoll, weil sich einerseits unter jedem Lochboden ein Gaspolster ausbildet, das den festen und flüssigen Bestandteilen des Reaktionsgemisches als Reaktionsraum verloren geht, und andererseits die zusätzliche Verbesserung des Verweilzeitspektrums minimal ist.

Die Löcher der Lochböden weisen im allgemeinen einen Durchmesser von maximal 20 mm, vorzugsweise von 2 bis 10 mm auf, wobei die Anzahl der Bohrungen in Abhängigkeit vom Durchsatz vorzugsweise so gewählt wird, daß eine Rückvermischung des von unten nach oben aufsteigenden Reaktionsgemischs zwischen den einzelnen Kammern weitgehend verhindert wird.

Bei dem in der Kolonne aufsteigenden Reaktionsgemisch handelt es sich um ein Gemisch aus flüssigen Bestandteilen (Lösungen der Ausgangsmaterialien und der sich bildenden Isocyanate), gasförmigen Bestandteilen (Phosgen und sich bildender Chlorwasserstoff) und (zumindest zu Beginn der Reaktion) festen Bestandteilen (im Lösungsmittel suspendierte Carbamylchloride bzw. Amin-hydrochloride). Optimale Reaktionsbedingungen liegen dann vor, wenn die Geschwindigkeit der aufsteigenden Gasphase in den Löchern der Lochböden bei 2 bis 20, vorzugsweise 3,5 bis 10 m/sec und die Geschwindigkeit der aufsteigenden flüssigen Phase in den Löchern der Lochböden bei 0,05 bis 0,4, vorzugsweise 0,1 bis 0,2 m/sec liegen.

Die Temperatur des, die Mischapparatur verlassenden, Reaktionsgemischs liegt im allgemeinen bei 40 bis 100°C, während die Temperatur am Kopf der Reaktionskolonne unterhalb 150°C, vorzugsweise bei 70 bis 130, insbesondere bei 90 bis 125°C liegt. Dies wird im allgemeinen durch geeignete Beheizung der Reaktionskolonne erreicht. Um das notwendige Phosgeniervolumen zu minimieren, ist es vorteilhaft, die zum Erreichen der gewünschten Überlauftemperatur erforderliche Energie im unteren Bereich des Phosgenierturms oder auch bereits vor Eintritt in den Reaktor einzubringen. Dadurch wird vermieden, daß ein Teil des Reaktorvolumens wegen zu niedriger Temperatur und daher zu langsamer Bruttoreaktionsgeschwindigkeit ineffektiv ist.

Die Abmessungen der Reaktionskolonne, sowie die Konstruktion der Böden und die Menge des in die Kolonne unten eingebrachten Reaktionsgemischs werden im übrigen im allgemeinen so bemessen, daß die mittlere Verweilzeit des Reaktionsgemischs in der Reaktionskolonne bei maximal 120, vorzugsweise maximal 60 Minuten liegt.

Der Druck am Kopf der Reaktionskolonne liegt im allgemeinen bei 1,2 bis 3, vorzugsweise 1,5 bis 2,5 bar (abs.). Es kann jedoch auch unter höheren oder niedrigeren Drücken gearbeitet werden.

Das die Reaktionskolonne am oberen Ende verlassende, flüssige und gasförmige Bestandteile enthaltende Reaktionsgemisch wird in an sich bekannter Weise zunächst von gasförmigen Bestandteilen (überschüssiges Phosgen und Chlorwasserstoff) befreit und anschließend destillativ aufgearbeitet.

Die in den nachfolgenden Beispielen gemachten Prozentangaben beziehen sich auf Gewichtsprozente.

## Beispiele

In den nachfolgenden Beispielen wurden die Mischungen der Diaminlösungen mit den Phosgenlösungen in einer entsprechend DE-AS 2 153 268 modifizierten Kreiselpumpe vorgenommen. Die so erhaltenen Lösungs-Suspensionen wurden mit einer Temperatur von 70-90°C in den unteren Teil der jeweiligen Reaktionsapparatur eingebracht. Als Reaktionsapparaturen dienten einerseits eine konventionelle Reaktionskaskade bestehend aus 3 hintereinandergeschalteten Reaktoren (Reaktionskolonnen) ohne Einbauten mit einem Gesamtvolumen von 17 m$^3$ in Analogie zu Ullmanns Encyclopädie der technischen Chemie, 4. Auflage (1977), Abbildung 2 auf Seite 352 (Vergleichsbeispiele 1a bis 5a) als auch eine erfindungsgemäß mit 23 Lochböden ausgerüstete Reaktionskolonne mit einem Gesamtvolumen von 7 m$^3$, wobei die Löcher einen Durchmesser von 10 mm aufwiesen (erfindungsgemäße Beispiele 1b bis 5b).

In allen Beispielen wurde ein Gemisch aus 65% 2,4-Diaminotoluol und 35% 2,6-Diaminotoluol (TDA) als Ausgangsamin verwendet.

Das in allen Beispielen am Kopf des (letzten) Reaktors unter einem Druck von ca. 1,5 bar (abs.) anfallende Reaktionsgemisch wurde jeweils von gasförmigen Bestandteilen befreit und anschließend destillativ aufgearbeitet.

## Beispiel 1a (Vergleich)

550 kg TDA/h, gelöst in 7300 kg/h o-Dichlorbenzol (ODB) werden mit 7070 kg/h einer 48%igen Phosgenlösung in ODB vermischt und die so erhaltene Lösungs-Suspension in die beheizte Reaktionskaskade gemäß Stand der Technik eingetragen, wobei die Temperaturführung so gewählt wurde, daß die Temperatur am Kopf des ersten Reaktors bei 80°C, am Kopf des zweiten Reaktors bei 125°C und am Kopf des dritten Reaktors bei 170°C lag. Bei einer mittleren Verweilzeit von 80 Minuten lag die Ausbeute an, dem Ausgangsamin entsprechendem, Diisocyanatotoluol-Isomerengemisch (TDI) bei 95,8%.

## Beispiel 1b (erfindungsgemäß)

Beispiel 1a wurde wiederholt, jedoch unter Verwendung der erfindungsgemäßen Reaktionskolonne (Phosgenierturm), die im unteren Teil so beheizt wurde, daß das am Kopf überlaufende flüssige Reaktionsgemisch eine Temperatur von ca. 115°C aufwies. Die Strömungsgeschwindigkeit der Gasphase in den einzelnen Löchern der Lochböden lag bei 6,6 m/sec, die der Flüssigphase bei ca. 0,18 m/sec.

Bei einer mittleren Verweilzeit von 33 Minuten wurde eine TDI-Ausbeute von 97,0% erzielt.

Beispiel 2a (Vergleich)

Beispiel 1a wurde wiederholt, jedoch unter Verwendung von lediglich 4450 kg/h ODB zum Lösen des Diamins.

Bei einer mittleren Verweilzeit von 100 Minuten wurde eine TDI-Ausbeute von 94,5% erzielt.

Beispiel 2b (erfindungsgemäß)

Beispiel 1b wurde wiederholt, jedoch unter Verwendung von lediglich 4450 kg/h ODB zum Lösen des Diamins. Die Strömungsgeschwindigkeit der Gasphase an den Löchern der Lochböden lag bei ca. 6,6 m/sec, die der Flüssigphase bei 0,14 m/sec. Bei einer mittleren Verweilzeit von 41 Minuten wurde eine TDI-Ausbeute von 96,5% erzielt.

Beispiel 3a (Vergleich)

Beispiel 1a wurde wiederholt, jedoch unter Verwendung von lediglich 3120 kg/h ODB zum Lösen des TDA. Nach einer mittleren Verweilzeit von 113 Minuten wurde eine TDI-Ausbeute von 93,3% erzielt.

Beispiel 3b (erfindungsgemäß)

Beispiel 1b wurde wiederholt, jedoch unter Verwendung von lediglich 3120 kg/h ODB zum Lösen des Diamins. Die mittlere Geschwindigkeit der Gasphase in den Löchern der Lochböden lag bei ca. 6,6 m/sec, die mittlere Geschwindigkeit der Flüssigphase bei ca. 0,12 m/sec. Nach einer mittleren Verweilzeit von 46 Minuten wurde eine TDI-Ausbeute von 95,7% erzielt.

Beispiel 4a (Vergleich)

Beispiel 1a wurde wiederholt, jedoch unter Verwendung von 400 kg/h TDA, 5300 kg/h ODB zum Lösen des TDA und von 5400 kg/h der 48%igen Phosgenlösung. Bei einer mittleren Verweilzeit von 107 Minuten wurde eine TDI-Ausbeute von 96,1% erzielt.

Beispiel 4b (erfindungsgemäß)

Die gleichen Mengenströme wie in Beispiel 4a ergaben unter Verwendung der erfindungsgemäßen Reaktionskolonne bei einer mittleren Verweilzeit von 44 Minuten eine TDI-Ausbeute von 97,0%. Die Geschwindigkeit der Gasphase in den Löchern der Lochböden lag hier bei ca. 4,8 m/sec, die der Flüssigphase bei ca. 0,13 m/sec.

Beispiel 5a (Vergleich)

Beispiel 1a wird wiederholt, jedoch unter Verwendung von 400 kg/h TDA, gelöst in 5300 kg/h ODB und von 2700 kg/h einer 48%igen Phosgenlösung. Bei einer mittleren Verweilzeit von 138 Minuten wurde eine TDI-Ausbeute von 94,6% erzielt.

Beispiel 5b (erfindungsgemäß)

Die gleichen Mengenströme wie in Beispiel 5a ergeben in der erfindungsgemäßen Kolonne bei einer mittleren Verweilzeit von 57 Minuten eine TDI-Ausbeute von 95,6%. Die Geschwindigkeit der Gasphase in den Löchern der Lochböden lag hierbei bei ca. 4,8 m/sec, die der Flüssigphase bei ca. 0,10 m/sec.

## Ansprüche

1. Verfahren zur kontinuierlichen Herstellung von organischen Mono- oder Polyisocyanaten in einstufiger Reaktion durch Umsetzung der in organischem Lösungsmittel gelösten, den Mono- oder Polyisocyanaten entsprechenden Mono- oder Polyamine mit in organischem Lösungsmittel gelöstem Phosgen bei erhöhter jedoch unterhalb 150°C liegenden Temperaturen, dadurch gekennzeichnet, daß man das durch kontinuierliche Vereinigung der Aminlösung mit der Phosgenlösung erhaltene Reaktionsgemisch kontinuierlich von unten nach oben eine Reaktionskolonne durchlaufen läßt, welche mindestens 10 durch Lochböden voneinander getrennte Kammern aufweist.

2. Verfahren gemäß Anspruch 1, dadurch gekennzeichnet, daß die Löcher der Lochböden einen Durchmesser von maximal 20 mm aufweisen.

3. Verfahren gemäß Anspruch 1 und 2, dadurch gekennzeichnet, daß die Geschwindigkeit der in der Kolonne aufsteigenden Gasphase in den Löchern der Lochböden bei 2 bis 20 m/sec und die Geschwindigkeit der aufsteigenden Flüssigphase in den Löchern der Lochböden bei 0,05 bis 0,4 m/sec liegt.

4. Verfahren gemäß Anspruch 1 bis 3 zur Herstellung von organischen Polyisocyanaten, dadurch gekennzeichnet, daß man als Ausgangsamine 2,4-Diaminotoluol, dessen technische Gemische mit 2,6-Diaminotoluol oder Polyamingemische der Diphenylmethanreihe verwendet.

5. Abänderung des Verfahrens gemäß Anspruch 1 bis 4, dadurch gekennzeichnet, daß man anstelle von einer mehrere, in Serie geschaltete, Lochbodenkolonnen mit insgesamt mindestens 10 Kammern verwendet.

## Claims

1. A process for the continuous preparation of organic mono- or polyisocyanates in a one-shot reaction by the reaction of mono- or polyamines corresponding to the mono-or polyisocyanates dissolved in an organic solvent with phosgene dissolved in an organic solvent at elevated temperatures below 150°C, characterised in that the reaction mixture obtained by continuously combining the amine solution with the phosgene solution is continuously passed from below upwards through a reaction column comprising at least 10 chambers separated by perforated plates.

2. A process according to Claim 1, characterised in that the perforations of the perforated plates have a diameter of at most 20 mm.

3. A process according to Claims 1 and 2, characterised in that the velocity of the gaseous phase ascending the column is from 2 to 20 m/sec in the perforations of the perforated plates and the velocity of the ascending liquid phase in the perforations of the perforated plates is from 0.05 to 0.4 m/sec.

4. A process according to Claims 1 to 3, for the preparation of organic polyisocyanates, characterised in that the starting amines used are 2,4-diaminotoluene, commercial mixtures thereof with 2,6-diaminotoluene or polyamine mixtures of the diphenylmethane series.

5. Modification of the process according to Claims 1 to 4, characterised in that instead of one column with perforated plates being used, a plurality of columns with perforated plates connected in series and having a total of at least 10 chambers is used.

## Revendications

1. Procédé de production continue de monoisocyanates ou de polyisocyanates organiques dans une réaction à une seule étape, par réaction des monoamines ou des polyamines correspondant aux monoisocyanates ou aux polyisocyanates, dissoutes dans un solvant organique, avec du phosgène dissous dans un solvant organique à des températures élevées mais inférieures à 150°C, caractérisé en ce qu'on fait passer le mélange réactionnel obtenu par réunion continue de la solution d'amine avec la solution de phosgène, de bas en haut à travers une colonne de réaction qui présente au moins 10 chambres séparées les unes des autres par des plateaux perforés.

2. Procédé suivant la revendication 1, caractérisé en ce que les trous des plateaux perforés ont un diamètre au maximum égal à 20 mm.

3. Procédé suivant les revendications 1 et 2, caractérisé en ce que la vitesse de la phase gazeuse montant dans la colonne dans les trous des plateaux perforés est de 2 à 20 m/s et la vitesse de la phase liquide ascendante dans les trous des plateaux perforés est de 0,05 à 0,4 m/s.

4. Procédé suivant les revendications 1 à 3 pour la production de polyisocyanates organiques, caractérisé en ce qu'on utilise comme amines de départ le 2,4-diaminotoluène, ses mélanges techniques avec le 2,6-diaminotoluène ou des mélanges de polyamines de la série du diphénylméthane.

5. Variante du procédé suivant les revendications 1 à 4, caractérisée en ce qu'on utilise, au lieu d'une seule colonne, plusieurs colonnes à plateaux perforés, montées en série, totalisant au moins 10 chambres.